# EUROPEAN PATENT APPLICATION

(11) **EP 2 332 662 A2**
(43) Date of publication of application: **15.06.2011**
(21) Application number: 10189398.0
(22) Date of filing: 29.10.2010
(51) Int. Cl.: B06B 1/06

(54) **Ultrasonic Diagnostic Probe and Method of Manufacturing the Same**

(30) Priority: 08.12.2009 KR 20090121164
(71) Applicant: MEDISON CO., LTD., Gangwon-do 250-875 (KR)
(72) Inventor: Min, Hae Kee, Ulsan-si (KR); Kim, Jae Yk, Seoul (KR); Lee, Sung Jae, Seoul (KR)
(74) Representative: Schmid, Wolfgang

(57) **Abstract**

An ultrasonic diagnostic probe and a method of manufacturing the same are disclosed. The probe includes a first piezoelectric member (142) having a first thickness, and a second piezoelectric member (144) having a second thickness and stacked on the first piezoelectric member (142). The method allows the production of several kinds of ultrasonic diagnostic probes, which generate ultrasound waves in different frequency bands, with a single kind of transducer module by changing connection between an external electrode and the electrodes of the probe.

## Description

### BACKGROUND OF THE INVENTION

### 1. Field of the Invention

The present invention relates to an ultrasonic diagnostic probe and, more particularly, to an ultrasonic diagnostic probe for generating internal images of a diagnosis target using ultrasound waves, and a method of manufacturing the same.

### 2. Description of the Related Art

Generally, an ultrasonic diagnostic apparatus refers to a non-invasive apparatus that irradiates an ultrasound signal from a surface of a patient body towards a target internal organ beneath the body surface and obtains an image of a monolayer or blood flow in soft tissue from information in the reflected ultrasound signal (ultrasound echo-signal). The ultrasonic diagnostic apparatus has been widely used for diagnosis of the heart, the abdomen, the urinary organs, and in obstetrics and gynecology due to various merits such as small size, low price, real-time image display, and high stability through elimination of radiation exposure, as compared with other image diagnostic systems, such as X-ray diagnostic systems, computerized tomography scanners (CT scanners), magnetic resonance imagers (MRIs), nuclear medicine diagnostic apparatuses, and the like.

The ultrasonic diagnostic apparatus includes a probe which transmits an ultrasound signal to a target and receives the ultrasound echo-signal reflected therefrom to obtain the ultrasound image of the target.

The probe includes a transducer, a case with an open upper end, a cover coupled to the upper end of the case to directly contact a body surface of a target, and the like.

To obtain a desired ultrasound image of the target, an operator moves the probe along the surface of the target or moves the probe while keeping the probe in contact with the surface of the target.

Ultrasound waves generated from the probe have a frequency band determined by the transducer, particularly, the characteristics of piezoelectric members therein. Thus, when producing several kinds of probes generating ultrasound waves in different frequency bands, it is necessary to produce transducers having different characteristics. Therefore, there is a need to solve such a problem.

### SUMMARY OF THE INVENTION

The present invention is conceived to solve the problem of the related art as described above, and an aspect of the invention is to provide an improved ultrasonic diagnostic probe that can be reconfigured into several kinds of probes through modification of a single kind of transducer therein, and a method of manufacturing the same.

In accordance with one aspect of the invention, an ultrasonic diagnostic probe includes: a first piezoelectric member having a first thickness; and a second piezoelectric member having a second thickness and stacked on the first piezoelectric member.

At least one of the first and second piezoelectric members may include electrodes.

The electrodes may be formed on both sides of each of the first and second piezoelectric members, respectively.

The first thickness may be different from the second thickness.

In accordance with another aspect of the invention, a method of manufacturing an ultrasonic diagnostic probe includes: forming electrodes on each of first and second piezoelectric members; stacking the first piezoelectric member on a backing; and stacking the second piezoelectric member on the first piezoelectric member.

The formation of electrodes may include forming the electrodes on both sides of each of the first and second piezoelectric members, respectively.

The method may further include connecting an external electrode to two of the electrodes formed on the first piezoelectric member and the electrodes formed on the second piezoelectric member.

The method may further include stacking a third piezoelectric member having electrodes on the first piezoelectric member after the formation of electrodes and before stacking the second piezoelectric member on the first piezoelectric member.

The method may further include connecting an external electrode to two of the electrodes formed on the first piezoelectric member, the electrodes formed on the second piezoelectric member, and the electrodes formed on the third piezoelectric member.

### BRIEF DESCRIPTION OF THE DRAWINGS

The above and other aspects, features and advantages of the invention will become apparent from the following description of embodiments given in conjunction with the accompanying drawings, in which:
- Fig. 1: is a schematic cross-sectional view of an ultrasonic diagnostic probe according to one embodiment of the present invention;
- Figs. 2 to 4: are cross-sectional views of a piezoelectric layer of the probe shown in Fig. 1;
- Fig. 5: is a flowchart of a method of manufacturing an ultrasonic diagnostic probe according to one embodiment of the present invention;
- Fig. 6: is a schematic cross-sectional view of an ultrasonic diagnostic probe according to another embodiment of the present invention;
- Fig. 7: is a cross-sectional view of a piezoelectric layer of the ultrasonic diagnostic probe shown in Fig. 6; and
- Fig. 8: is a flowchart of a method of manufacturing an ultrasonic diagnostic probe according to another embodiment of the present invention.

### DETAILED DESCRIPTION OF THE EMBODIMENT

Exemplary embodiments of the invention will now be described in detail with reference to the accompanying drawings. It should be noted that the drawings are not to precise scale and may be exaggerated in thickness of lines or size of components for descriptive convenience and clarity only. Furthermore, terms used herein are defined by taking functions of the invention into account and can be changed according to the custom or intention of users or operators. Therefore, definition of the terms should be made according to overall disclosures set forth herein.

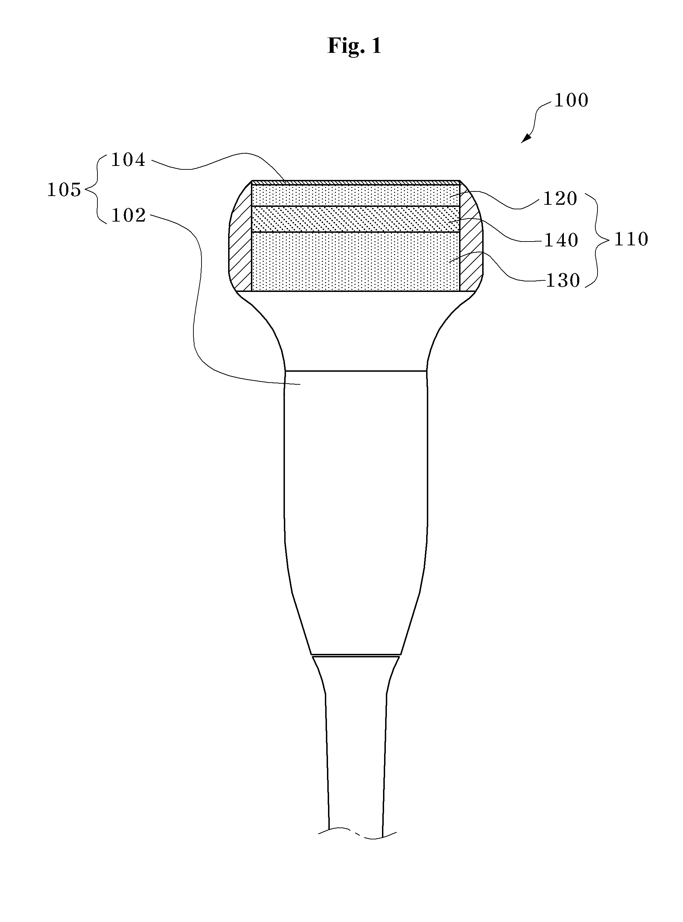

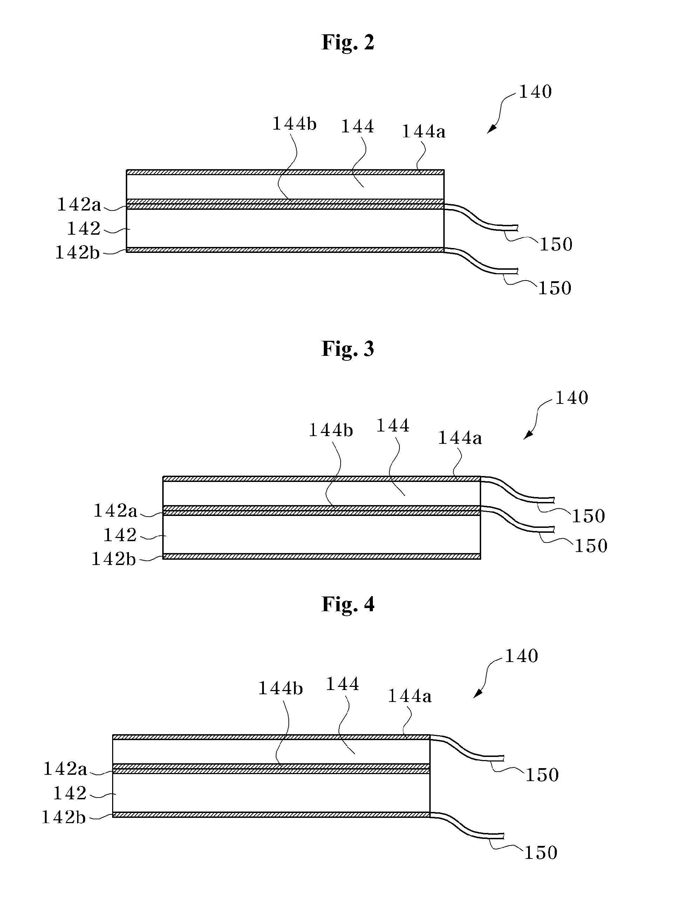

Fig. 1 is a schematic cross-sectional view of an ultrasonic diagnostic probe according to one embodiment and Figs. 2 to 4 are cross-sectional views of a piezoelectric layer of the ultrasonic diagnostic probe shown in Fig. 1.

Referring to Figs. 1 to 4, an ultrasonic diagnostic probe 100 of this embodiment includes a transducer 110.

The transducer 110 is disposed inside a case part 105. In this embodiment, the case part 105 includes a case 102 and a cover 104. The case 102 defines an external appearance of the ultrasonic diagnostic probe 100 and receives the transducer 110 therein. The case 102 is open at an upper end thereof, to which the cover 104 is coupled to be brought into direct contact with a surface of a diagnostic target.

The transducer 110 disposed inside the case part 105 includes a matching layer 120, a backing layer 130, a lens layer (not shown), and a piezoelectric layer 140.

The matching layer 120 reduces difference in sound impedance between the piezoelectric layer 140 and the target to allow ultrasound signals generated from the piezoelectric layer 140 to be efficiently transferred to the target. The matching layer 120 is disposed in front of the piezoelectric layer 140 and may be formed of a glass or resin material.

The backing layer 130 prevents image distortion by blocking unnecessary propagation of the ultrasound signals in the rearward direction of the piezoelectric layer 140. The backing layer 130 is disposed behind the piezoelectric layer 140 and may be formed of a material containing a rubber, to which epoxy, tungsten powder, and the like are added.

The lens layer focuses the ultrasound signals from the piezoelectric layer on a certain region. The lens layer is disposed in front of the matching layer 120.

In this embodiment, the piezoelectric layer 140 is disposed between the matching layer 120 and the backing layer 130, and converts an electrical signal into a sound signal or vice versa using vibration from piezoelectric members 142, 144. The piezoelectric layer 140 includes a first piezoelectric member 142 and a second piezoelectric member 144.

The first and second piezoelectric members 142, 144 generate ultrasound waves using a resonance phenomenon and may be formed of a ceramic of lead zirconate titanate (PZT), a PZNT single crystal made of a solid solution of lead zinc niobate and lead titanate, a PZMT single crystal made of a solid solution of lead magnesium niobate and lead titanate, or the like.

The first piezoelectric member 142 is formed to have a first thickness. The second piezoelectric member 144 is formed to have a second thickness. In this embodiment, the first thickness is different from the second thickness, so that the first and second piezoelectric members 142, 144 are formed to have different thicknesses. Each of the first and second piezoelectric members 142, 144 generates ultrasound waves in a predetermined frequency band.

In general, the peak frequency is inversely proportional to the thickness of a vibrating material, and is thus determined depending on the thickness of the material when all other conditions are the same. In other words, as the thickness of the material increases, the peak frequency is lowered, and as the thickness of the material decreases, the peak frequency is increased.

Accordingly, the first and second piezoelectric members 142, 144 having different thicknesses generate ultrasound waves that have different frequency bands. In this embodiment, the second piezoelectric member 144 is thinner than the first piezoelectric member 142, so that the frequency band of ultrasound waves generated from the second piezoelectric member 144 is higher than that of ultrasound waves from the first piezoelectric member 142.

At least one of the first and second piezoelectric members 142, 144 is provided with electrodes 142a, 142b, 144a, 144b. In this embodiment, the electrodes 142a, 142b, 144a, 144b are illustrated as being formed on both sides of the first and second piezoelectric members 142, 144, respectively. The electrodes 142a, 142b, 144a, 144b may be formed of a highly conductive material, such as gold, silver or copper.

One of the electrodes 142a, 142b formed on the first piezoelectric member 142 serves as a signal electrode (or positive electrode) of the first piezoelectric member 142 and the other serves as a ground electrode (negative electrode) of the first piezoelectric member 142. The electrodes 142a, 142b are formed such that the signal electrode is separated from the ground electrode. In this embodiment, the electrode 142a on a front side of the first piezoelectric member 142 is illustrated as the signal electrode, and the electrode 142b on a rear side of the first piezoelectric member 142 is illustrated as the ground electrode.

Further, one of the electrodes 144a, 144b formed on the second piezoelectric member 144 serves as a signal electrode (or positive electrode) of the second piezoelectric member 144 and the other serves as a ground electrode (negative electrode) of the second piezoelectric member 144. The electrodes 144a, 144b are formed such that the signal electrode is separated from the ground electrode. In this embodiment, the electrode 144a on a front side of the second piezoelectric member 144 is illustrated as the signal electrode, and the electrode 144b on a rear side of the first piezoelectric member 144 is illustrated as the ground electrode.

The first and second piezoelectric members 142, 144 having the electrodes 142a, 142b, 144a, 144b are stacked and connected to each other. For example, the second piezoelectric member 144 is stacked on the front side of the first piezoelectric member 142 and connected to the first piezoelectric member 142. Here, the electrode 142a formed on the front side of the first piezoelectric member 142 is connected to the electrode 144b formed on the rear side of the second piezoelectric member 144, so that the first piezoelectric member 142 is electrically connected to the second piezoelectric member 144 via the connection between the electrodes 142a and 144b.

At least one of the first and second piezoelectric members 142, 144 having the electrodes 142a, 142b, 144a, 144b is connected to an external electrode 150. In this embodiment, the external electrode is illustrated as a wire electrode provided to a printed circuit board (PCB).

According to this embodiment, the external electrode 150 is connected to two of the electrodes 142a, 142b on the first piezoelectric member 142 and the electrodes 144a, 144b on the second piezoelectric member 144. In one embodiment, the external electrode 150 may be connected to the two electrodes 142a, 142b of the first piezoelectric member 142 (see Fig. 2). In another embodiment, the external electrode 150 may be connected to the two electrodes 144a, 144b of the second piezoelectric member 144 (see Fig. 3). In a further embodiment, the external electrode 150 may be connected to the electrode 144a formed on the front side of the second piezoelectric member 144 and the electrode 142b formed on the rear side of the first piezoelectric member 142 (see Fig. 4).

As a result, the piezoelectric layer 140 allows variation in range of a vibrating region depending on connection positions of the external electrode 150 thereto, so that the frequency band of ultrasound signals generated therefrom varies.

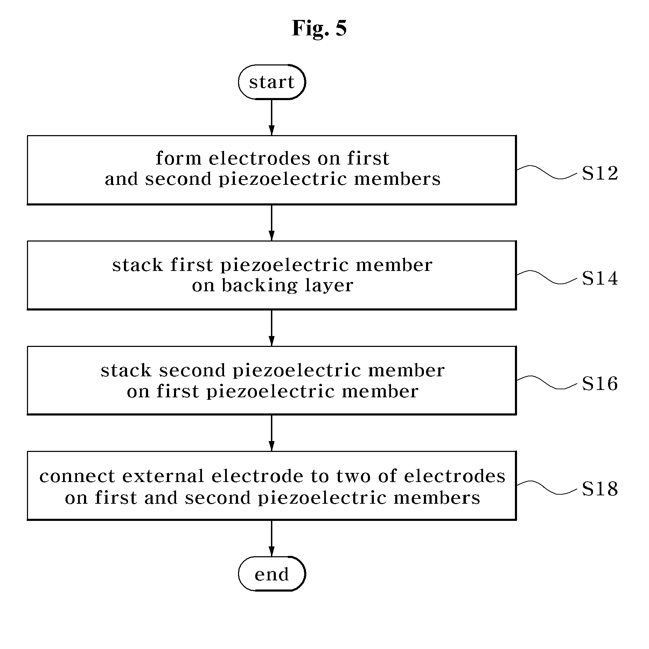

Fig. 5 is a flowchart of a method of manufacturing the ultrasonic diagnostic probe according to the embodiment of the present invention.

Next, a method of manufacturing the ultrasonic diagnostic probe according to this embodiment will be described with reference to Figs. 1 to 5.

Referring to Figs. 1 to 5, in order to fabricate the ultrasonic diagnostic probe 100 of this embodiment, electrodes 142a, 142b, 144a, 144b are formed on first and second piezoelectric members 142, 144, respectively, in S12. The electrodes 142a, 142b, 144a, 144b are formed on both sides of the first and second piezoelectric members 142, 144, respectively, such that each signal electrode is separated from each ground electrode.

Then, the first piezoelectric member 142 is stacked on a backing layer 130 in S14, and the second piezoelectric member 144 is stacked on the first piezoelectric member 142 in S16. Here, the first piezoelectric member 142 is electrically connected to the second piezoelectric member 144 via the electrodes 142a, 144b.

With the second piezoelectric member 144 stacked on the first piezoelectric member 142, an external electrode 150 is connected to two of the electrodes 142a, 142b on the first piezoelectric member 142 and the electrodes 144a, 144b on the second piezoelectric member 144, in S18. The connection between the external electrode 150 and the electrodes 142a, 142b, 144a, 144b may be achieved using an anisotropic conductor or a soldering material, such as lead and the like. The connection method is apparent to those skilled in the art and a detailed description thereof will thus be omitted herein.

The connection between the external electrode 150 and the electrodes 142a, 142b, 144a, 144b may be achieved in any form of a connection between the external electrode 150 and the two electrodes 142a, 142b of the first piezoelectric member 142, a connection between the external electrode 150 and the two electrodes 144a, 144b of the second piezoelectric member 144, a connection between the external electrode 150 and the electrodes 144a and 142b respectively formed on the front side of the second piezoelectric member 144 and the rear side of the first piezoelectric member 142, and the like.

According to this embodiment, the piezoelectric layer 140 and the transducer 110 including the same provide different frequency bands of ultrasound signals depending on the connection form between the external electrode 150 and the electrodes 142a, 142b, 144a, 144b.

With such a single kind of transducer 110, a different kind of ultrasonic diagnostic probe 100 capable of generating ultrasound waves in different frequency bands may be fabricated simply by changing the connection form between the external electrode 150 and the electrodes 142a, 142b, 144a, 144b.

Further, the ultrasonic diagnostic probe 100 includes the piezoelectric layer 140 formed by stacking the plural piezoelectric members 142, 144 generating ultrasound signals in a high frequency band, thereby enhancing RF characteristics and quality of ultrasound images.

On the other hand, although the first and second piezoelectric members 142, 144 are illustrated as having different thicknesses in this embodiment, it should be noted that the invention is not limited thereto. In another embodiment, the first and second piezoelectric members 142, 144 may have the same thickness, and even in this case, the transducer 140 generates ultrasound signals in different frequency bands depending on the connection between the external electrode 150 and the electrodes 142a, 142b, 144a, 144b.

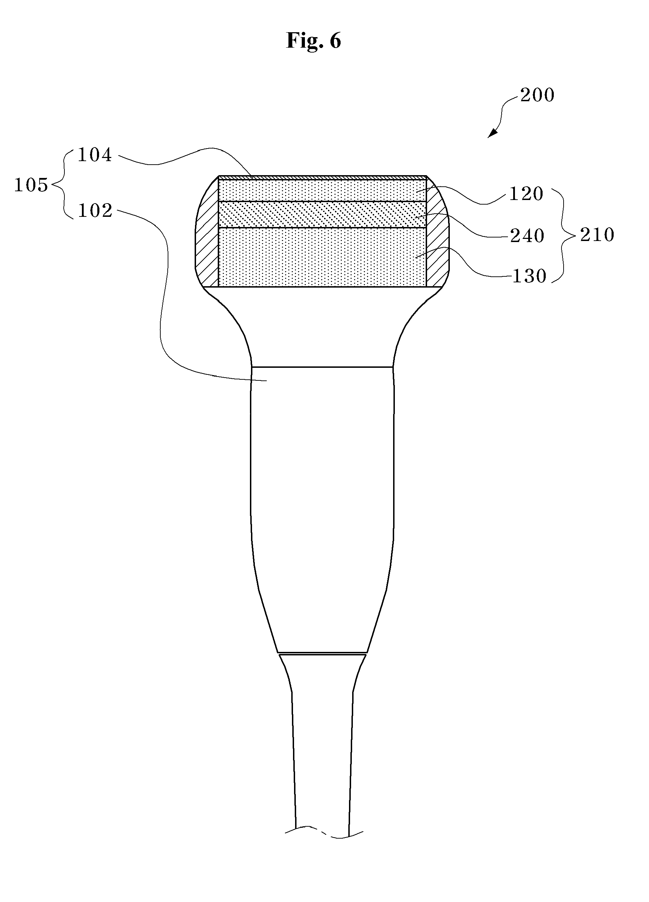

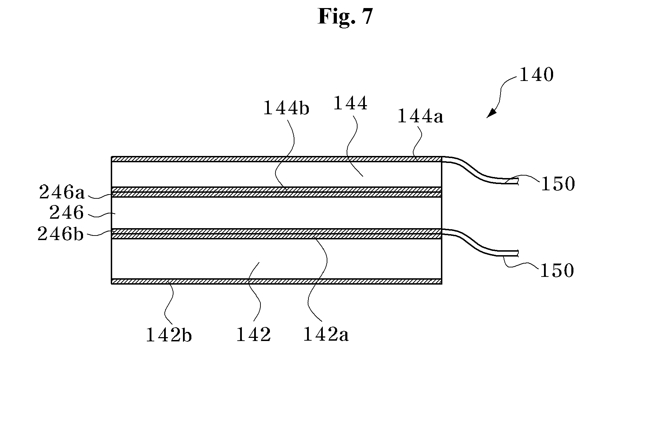

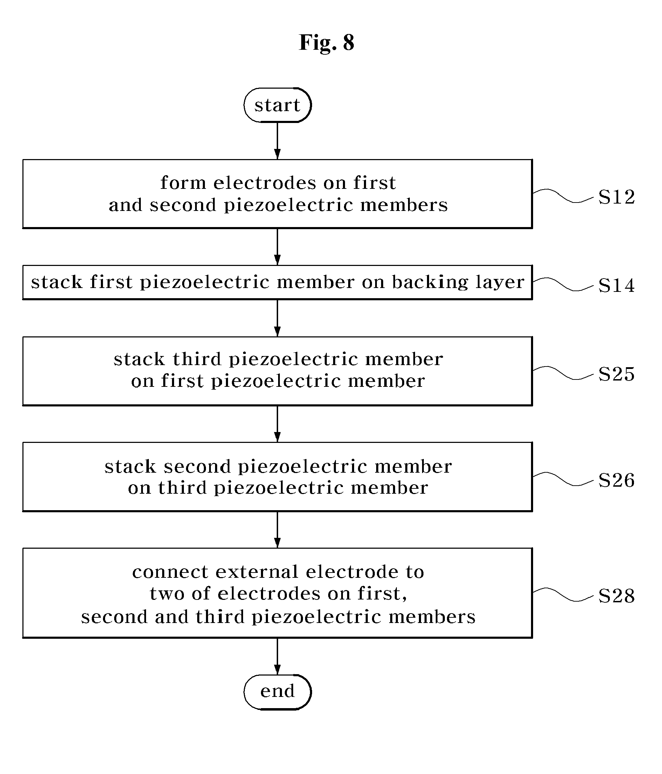

Fig. 6 is a schematic cross-sectional view of an ultrasonic diagnostic probe according to another embodiment, Fig. 7 is a cross-sectional view of a piezoelectric layer of the ultrasonic diagnostic probe shown in Fig. 6, and Fig. 8 is a flowchart of a method of manufacturing the ultrasonic diagnostic probe according to this embodiment.

For descriptive convenience, the same or similar components of the embodiment as those of the above embodiment will be denoted by the same reference numerals, and a detailed description thereof will be omitted herein.

Referring to Figs. 6 and 7, an ultrasonic diagnostic probe 200 of this embodiment includes a transducer 210, which includes a piezoelectric layer 240 including first to third piezoelectric members 142, 144, 246.

The third piezoelectric member 246 generates ultrasound waves in a predetermined frequency band using a resonance phenomenon, as in the first and second piezoelectric members 142, 144.

The third piezoelectric member 246 may have the same thickness as one of the first and second piezoelectric members 142, 144 or may have a different thickness from those of the first and second piezoelectric members 142, 144. In this embodiment, the third piezoelectric member 246 is illustrated as having a different thickness from those of the first and second piezoelectric members 142, 144. The third piezoelectric member 246 is also provided with electrodes 246a, 246b as in the first and second piezoelectric members 142, 144 .

The third piezoelectric member 246 is stacked between the first and second piezoelectric members 142, 144. The third piezoelectric member 246 is electrically connected to the first piezoelectric member 142 via electrodes 142a, 246b and to the second piezoelectric member 144 via electrodes 144b, 246a.

According to this embodiment, an external electrode 150 is connected to two of the electrodes 142a, 142b on the first piezoelectric member 142, the electrodes 144a, 144b on the second piezoelectric member 144, and the electrodes 246a, 246b on the third piezoelectric member 246. As in the piezoelectric layer 140 (see Fig. 2) of the above embodiment, the piezoelectric layer 240 allows variation in range of a vibrating region depending on connection positions of the external electrode 150 thereto, so that the frequency band of ultrasound signals generated therefrom varies.

Next, a method of manufacturing the ultrasonic diagnostic probe according to this embodiment will be described with reference to Figs. 6 to 8.

Referring to Figs. 6 to 8, in order to produce the ultrasonic diagnostic probe 200 of this embodiment, electrodes 142a, 142b, 144a, 144b are formed on first and second piezoelectric members 142, 144, respectively, in S12, and electrodes 246a, 246b are formed on a third piezoelectric member 246.

Then, the first piezoelectric member 142 is stacked on a backing layer 130 in S14, the third piezoelectric member 246 is stacked on the first piezoelectric member 142 in S25, and the second piezoelectric member 144 is stacked on the third piezoelectric member 246 in S26. The first to third piezoelectric members 142, 144, 246 are electrically connected to one another via the electrodes 142a, 144b, 246a, 246b.

With the first to third piezoelectric members 142, 144, 246 stacked on the backing layer, an external electrode 150 is connected to two of the electrodes 142a, 142b on the first piezoelectric member 142, the electrodes 144a, 144b on the second piezoelectric member 144, and the electrodes 246a, 246b on the third piezoelectric member 246, in S28.

As in the piezoelectric layer 140 (see Fig. 2) and the transducer 110 (see Fig. 1) of the above embodiment, the piezoelectric layer 240 and the transducer 210 including the same allow variation in range of a vibrating region depending on connection positions of the external electrode 150 thereto, so that the frequency band of ultrasound signals generated therefrom varies.

Although this embodiment is illustrated as having a single third piezoelectric member 246 between the first and second piezoelectric members 142, 144, it should be noted that the invention is not limited thereto and various modifications can be realized. In an alternative embodiment, a plurality of third piezoelectric members 246 having the same or different thicknesses may be stacked between the first and second piezoelectric members 142, 144.

As such, according to the embodiments, several kinds of ultrasonic diagnostic probes generating ultrasound waves in different frequency bands may be manufactured using a single kind of transducer module by changing connection between an external electrode and electrodes of the probe.

Further, the ultrasonic diagnostic probe according to the embodiment includes a piezoelectric layer formed by stacking piezoelectric members generating ultrasound signals in a high frequency band, thereby enhancing RF characteristics and quality of ultrasound images.

Although some embodiments have been provided to illustrate the invention in conjunction with the drawings, it will be apparent to those skilled in the art that the embodiments are given by way of illustration only, and that various modifications and equivalent embodiments can be made without departing from the spirit and scope of the invention. The scope of the invention should be limited only by the accompanying claims.

## Claims

1. An ultrasonic diagnostic probe, **characterized by** comprising:
a first piezoelectric member (142) having a first thickness; and
a second piezoelectric member (144) having a second thickness and stacked on the first piezoelectric member (142).

2. The ultrasonic diagnostic probe according to claim 1, **characterized in that** at least one of the first and second piezoelectric members (142, 144) comprises electrodes (142a, 142b, 144a, 144b).

3. The ultrasonic diagnostic probe according to claim 2, **characterized in that** the electrodes (142a, 142b, 144a, 144b) are formed on both sides of each of the first and second piezoelectric members (142, 144), respectively.

4. The ultrasonic diagnostic probe according to any one of claims 1 to 3, **characterized in that** the first thickness is different from the second thickness.

5. A method of manufacturing an ultrasonic diagnostic probe, **characterized by** comprising:
forming electrodes (142a, 142b, 144a, 144b) on each of first and second piezoelectric members (142, 144);
stacking the first piezoelectric member (142) on a backing layer (130); and
stacking the second piezoelectric member (144) on the first piezoelectric member (142).

6. The method according to claim 5, **characterized in that** the formation of electrodes (142a, 142b, 144a, 144b) comprises forming the electrodes (142a, 142b, 144a, 144b) on both sides of each of the first and second piezoelectric members (142, 144), respectively.

7. The method according to claim 5 or 6, **characterized by** further comprising:
connecting an external electrode (150) to two of the electrodes (142a, 142b) formed on the first piezoelectric member (142) and the electrodes (144a, 144b) formed on the second piezoelectric member (144).

8. The method according to claim 5 or 6, **characterized by** further comprising:
stacking a third piezoelectric member (246) having electrodes (246a, 246b) on the first piezoelectric member (142) after the formation of electrodes (142a, 142b, 144a, 144b) and before stacking the second piezoelectric member (144) on the first piezoelectric member (142).

9. The method according to claim 8, **characterized in that** further comprising:
connecting an external electrode (150) to two of the electrodes (142a, 142b) formed on the first piezoelectric member (142), the electrodes (144a, 144b) formed on the second piezoelectric member (144), and the electrodes (246a, 246b) formed on the third piezoelectric member (246).
